Europäisches Patentamt

European Patent Office

Office européen des brevets

(11) Veröffentlichungsnummer: **0 191 427 B1**

## EUROPÄISCHE PATENTSCHRIFT

(45) Veröffentlichungstag der Patentschrift: **29.04.92**

(21) Anmeldenummer: **86101585.7**

(22) Anmeldetag: **07.02.86**

(51) Int. Cl.⁵: **A61F 2/10**

Die Akte enthält technische Angaben, die nach dem Eingang der Anmeldung eingereicht wurden und die nicht in dieser Patentschrift enthalten sind.

(54) **Haarprothese.**

(30) Priorität: **14.02.85 IT 1951385**

(43) Veröffentlichungstag der Anmeldung:
**20.08.86 Patentblatt 86/34**

(45) Bekanntmachung des Hinweises auf die Patenterteilung:
**29.04.92 Patentblatt 92/18**

(84) Benannte Vertragsstaaten:
**AT BE CH DE FR GB IT LI LU NL SE**

(56) Entgegenhaltungen:
**DE-A- 2 912 043**
**FR-A- 2 372 621**
**GB-A- 2 045 089**
**US-A- 4 216 777**

(73) Patentinhaber: **SALC di Pellegrino & C. SDF**
**Corso Martiri della Libertà**
**I-17014 Cairo Montenotte(IT)**

(72) Erfinder: **Santi, Claudio**
**Via Mignone 37/25**
**I-17100 Savona(IT)**

(74) Vertreter: **Mayer, Hans Benno, Dipl.-Ing.**
**de Dominicis & Mayer Piazzale Marengo 6**
**I-20121 Milano(IT)**

EP 0 191 427 B1

**Beschreibung**

Die vorstehende Erfindung betrifft eine Haarprothese, die geeignet ist, ein teilweises oder vollstaendiges Haarimplantat herzustellen.

Es sind bereits verschiedene Verfahren bekannt geworden, mit denen versucht wird, Teilglatzen oder totale Glatzen zu beseitigen. Diese bekannten Verfahren haben jedoch den Nachteil, dass sie entweder sehr kostenaufwendig sind, dass das Endergebnis nicht natuerlich erscheint oder aber, dass das erzielbare Ergebnis nicht ueber einen langen Zeitraum bestaendig ist.

Es ist z.B. aus der italienischen Patentanmeldung 68405 A/82 eine Prothese zur Herstellung eines Haarimplantates bekannt geworden, bei dem ein aus inertem Material bestehendes Fadenstueck zum Einsatz gelangt, das an jedem Ende mit einem Haarbueschel verbunden ist, wobei jedes Haarbueschel mit dem Fadenende fest verbunden ist. Durch einen chirurgischen Eingriff werden diese Haarbueschel derart in die Kopfhaut eingesetzt, dass der meist aus synthetischem Material bestehende Faden in der Kopfhaut zu liegen kommt und als Verankerung dient, wogegen die an den Enden des Haltefadens befestigten Haarschueschel aus der Kopfhaut abstehen. Diese bekannte Haarprothese ist nicht nur sehr aufwendig, was den chirurgischen Eingriff anbetrifft, sondern das Endergebnis des Implantats ermoeglicht es nicht, eine Haarprothese herzustellen, die natuerlichen Eindruck erweckt.

Es ist ferner bekannt, einzelne Haare in die Kopfhaut einzusetzen, Die verwendeten Haare bestehen aus Kunststoff. In diesem Fall koennen teilweise Vergiftungen und Reizungen der Kopfhaut auftreten, die zu einer Entzuendung des Gewebes fuehren, besonders aufgrund der Farbstoffe, die in den Haaren aus Kunststoffmaterial fuer die Prothese enthalten sind. Diese kleinen Farbstoffmengen befreien sich unaufhaltsam im Laufe der Zeit und verursachen die genannten Reizungen der Kopfhaut.

Aus der Vorveroeffentlichung DE-A-29 12 043 ist eine Prothese, bestehend aus einem haarartigen Faden zum Einpflanzen in das Gewebe der Kopfhaut bekanntgeworden. Bei dieser Prothese werden sowohl natürlich als auch nicht natuerliche Haare, nämlich Faeden aus Kunststoff, z. B. aus polymerem Material, eingepflanzt. Bei künstlichen Haaren treten somit die bereits erwaehnten Nachteile auf. So kann es z. B. zu Unvertraeglichkeit und Entzuendung der Kopfhaut kommen. Es ist jedoch nicht moeglich, die einstueckig ausgebildete ringartige Wurzel bei entzuendeter Kopfhaut aufzuloesen (reversible Schlinge), um die Haarprothese aus der Kopfhaut durch Aufbringen einer Zugkraft zu entfernen. In den meisten Faellen ist bei derartigen Implantaten ein Entfernen der Haarprothese nur auf chirurgischem Wege moeglich.

In der GB-A-2 045 089 wird eine Haarprothese beschrieben, bei der am Wurzelende der einzusetzenden Haare eine einfache Schlaufe gebildet wird, die die Form des griechischen Buchstaben Gamma einnimmt. Die Haarwurzel wird mit einer Kunststoffschicht ummantelt und durch festes Verkleben oder Verschweissen der sich kreuzenden Haarstraenge wird eine Endschlaufe gebildet, die eine abstehende Verlaengerung aufweist, die zur Verankerung des Implantats in der Kopfhaut dient.

Die Verschweissung oder Verklebung der Haarstraenge unter einem spitzen Winkel fuehrt zum Haarausriss schon bei geringer mechanischer Belastung. Das abstehende Haarende erlaubt kein reversibles Aufloesen der Wurzelschlinge im Fall einer Entzuendung. Die gebildete Schlinge des gammafoermigen Endstueckes weist eine sehr kleine tropfenartige Durchgangsoeffnung auf, die nur in den guenstigsten Faellen waehrend des Vernarbungsprozesses von der Kopfhaut durchdrungen werden kann.

Es ist Aufgabe der vorstehenden Erfindung, eine neuartige Prothese zur Erstlelung eines teilweisen oder vollstaendigen Haarimplantates vorzuschlagen, die die Nachteile des Standes der Technick nicht aufweist, die es ferner ermoeglicht, das Implantat schnell und einfach in die Kopfhaut einzusetzen und ferner die Moeglichkeit eroeffnet, sich an alle individuellen Beduerfnisse des Traegers der Haarprothese anzupassen und besonders eine Haarprothese zu schaffen, die besonders die Verwendung natuerlicher Haare er erlaubt, die nicht eingefaerbt sind und auf keinen Fall zu toxologischen Nebenwirkungen fuehren.

Diese Aufgaben werden erfindungsgemaess dadurch geloest, dass eine Haarprothese zur Herstellung eines teilweisen oder vollstaendigen Haarersatzes mittels Implantats aus Naturhaaren Verwendung findet, die sich dadurch kennzeichnet, dass ein oder mehrere Naturhaare an ihrem in die Kopfhaut einzupflanzenden, der natuerlichen Wurzel der Haare entsprechenden, Ende zu einem ringfoermigen Gebilde zusammengefuegt sind und diesem durch gegenseitiges Umwickeln und/oder Verflechten und anschliessendes Aufbringen eines Inertklebers und Abschneiden der abstehenden Endstuecke der Haarenden die Gestalt eines glatten, steif-elastischen Ringes verliehen wird, wobei das ringfoermige Wurzelende durch Aufbringen eines Inertklebers gefestigt wird.

In vorteilhafter Weise besteht der Kleber aus Zyan-Acrylat.

Es ist ferner vorteilhaft, dass das Haarbueschel aus natuerlichen Haaren mit unterschiedlicher Faerbung und unterschiedlicher Haardicke besteht.

Der Durchmesser des ringfoermigen Wurzelendes weist in vorteilhafter Weise einem Wert von 0,5 - 2 mm auf.

Weitere Vorteile und besondere Ausfuehrungsformen der Erfindung koennen der nun folgenden Beschreibung, den Unteranspruechen und den beigefuegten Zeichnungen entnommen werden.

Ein Ausfuehrungsbeispiel der Erfindung wird nun genauer beschrieben und in den beigefuegten Zeichnungen dargestellt:

Fig. 1 zeigt in Ansicht ein Haarbueschel, bestehend aus vier Einzelhaaren ohne Endwurzel;

Fig. 2 zeigt das Haarbueschel nach Fig. 1, mit Darstellung des Vorganges zur Herstellung einer ringfoermigen Endwurzel;

Fig. 3 zeigt das Aufbringen eines Inertklebers, mit dem die verschlungenen oder verflochtenen Haarenden in ihrer Lage fixiert werden;

Fig. 4 zeigt schematisch das Abschneiden der Endhaare, die von dem ringfoermigen, wurzelartigen, steif-elastischen Endstueck abstehen;

Fig. 5 zeigt schematisch den Einsetzvorgang des wurzelartigen Endstueckes des Haarimplantates in die Kopfhaut, unter Verwendung einer mit hakenfoermigem Ende versehenen Nadel; und

Fig. 6 zeigt in vergroesserter Darstellung im Querschnitt das erfindungsgemaesse Haarimplantat, eingesetzt in die Hopfhaut des Traegers.

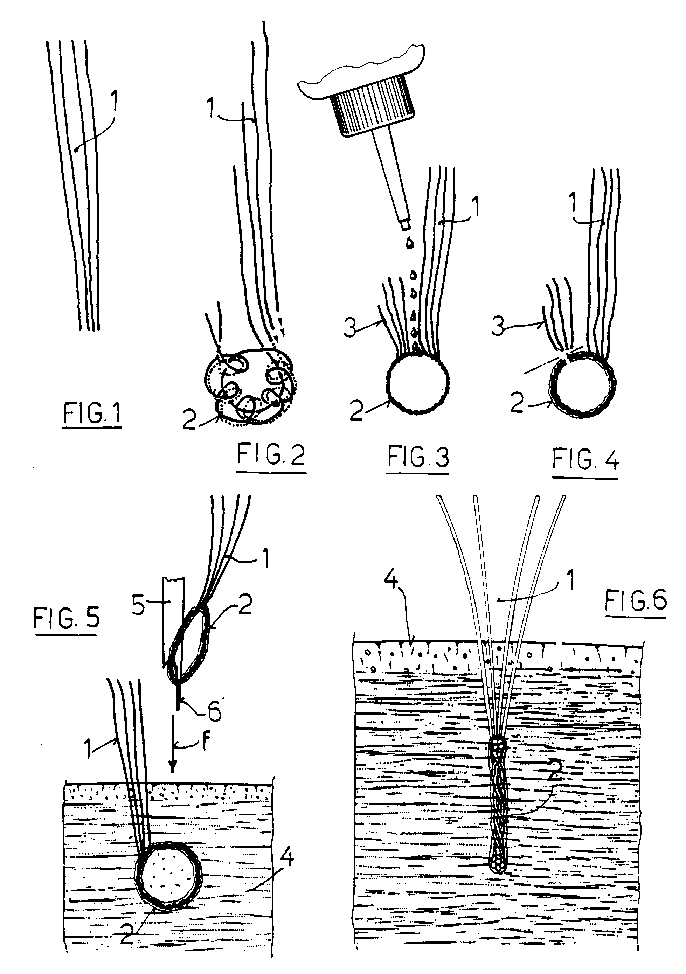

Wie der Fig. 1 zu entnehmen ist, wird eine Anzahl von Haaren, die sich von einem, zwei bis auf z.B. zehn Einzelhaaren aendern kann, in paralleler Anordnung zusammengefuegt. Die natuerlichen Haare 1 werden derartig zusammengefuegt, dass ihre Keratinschuppen in der gleichen Art wie die verbleibenden, natuerlichen Haare des Prothesetraegers angeordnet sind. Selbstverstaendlich unterliegt die Auswahl der Haarfarbe, sowie die Anzahl der Haare fuer die Bildung des Haarbueschels Veraenderungen, die vom gewuenschten Implantatergebnis abhaengig sind, besonders unter Beruecksichtigung, ob eine mehr oder weniger dichte Anordnung des Implantats gewuenscht ist. Weiter ist diese Auswahl vom Ort, an dem das Implantat einzusetzen ist, d.h. in Abhaengigkeit, ob das Haarbueschel z.B. in der Stirngegend, in der Schlaefengegend oder an besonders deutlich erkennbaren Teilen des Kopfes anzuordnen ist. Um das Endergebnis des Implantats noch echter und naturgetreuer erscheinen zu lassen, besteht z.B. die Moeglichkeit, fuer ein Haarbueschel Haare mit unterschiedlicher Toenung und unterschiedlicher Haarstaerke vorzusehen, wobei im Haarbueschel auch vereinzelt graue Haare enthalten sein koennen. Diese Tatsache laesst das Endergebnis wesentlich naturgetreuer und aehnlich dem natuerlichen Wuchs der Haare erscheinen.

Wie der Fig. 2 zu entnehmen ist, werden die Haare 1 an ihrem Ende gegenseitig umschlungen oder verflochten, um dieses Ende aehnlich der Wurzel natuerlicher Haare auszubilden. Es wird somit ein Wurzelersatz geschaffen, der in vorteilhafter Weise die Form eines kleinen Kreisringes 2 aufweist. Dieser Kreisring 2 weist in vorteilhafter Weise einen variablen Durchmesser von 0,5 - 2 mm auf. Die Abmessungen des Endringes 2, der den Wurzelersatz darstellt, ist im wesentlichen abhaengig von der Beschaffenheit der Kopfhaut des Prothesetraegers. Die Kopfhaut kann mehr oder weniger dick ausgebildet sein. Wie bereits erwaehnt, sind die Haare 1 des Implantats so angeordnet, dass die natuerliche Anordnung der Keratinschuppen des Implantattraegers beruecksichtigt wird.

Um das ringfoermige, aus verflochtenen Haarenden bestehende Endstueck fest in der Kopfhaut anzuordnen und, um gleichzeitig einen kleinen Endring mit elastisch steifen Merkmalen herstellen zu koennen, werden, wie der Fig. 3 zu entnehmen ist, auf dem verflochten Endring ein oder mehrere Tropfen eines Inertklebers aufgebracht. Wie schon ausgefuehrt, finden zu diesem Zwecke in vorteilhafter Weise Inertkleber Verwendung, die bereits in der Chirurgie, sowie zur Herstellung chirurgischer Prothesen bekannt sind. Diese Kleber sind z.B. unter den Handelsnamen "Histoacryl" oder "Kemicyak" im Handel bekannt. Unter Aufbringung des Klebers in Form von einigen wenigen Tropfen, wie in Fig. 3 dargestellt, erfolgt aufgrund der Kapillarwirkung der Verflechtung eine vollstaendige Benetzung des Endringes 2, der den prothetischen Wurzelersatz darstellt, mit dem Kleber. Nach Aushaerten des Klebers wird somit ein Endring 2 hergestellt, der steif-elastische Merkmale aufweist. Dieses besondere Merkmal des Ringes 2 ist von grosser Wichtigkeit fuer das Einfuehren in die, sowie das Befestigen der Haarprothese 1, 2 in der Kopfhaut des Prothesentraegers. Es ist jedoch zu unterstreichen, dass lediglich der Ring 2, der das Endstueck des Haarbueschels bildet, steif-elastische Merkmale aufweist, wogegen die vom Wurzelring 2 abzweigenden Haare des Gesamtbueschels sich in natuerlicher und vollkommen freier Anordnung vom Endring 2 verzweigen.

In vorteilhafter Weise besteht das Haarbueschel aus 3 - 10 Haaren, die derartig zusammengefasst sind, dass ein einziger Wurzelersatz 2 gebildet wird. Natuerlich koennen auch nur Einzelhaare Verwendung finden und mit einem Endring 2 versehen werden.

Nach Aushaerten des Klebers, wie der Fig. 4 zu entnehmen ist, werden die abstehenden Endstuecke 3 der verflochtenen Haare 1 abgeschnitten, um einen einwandfreien, glatten, kreisringartigen Wurzelersatz ohne stoerende, abstehende Haare zu bilden.

Wie der Fig. 5 zu entnehmen ist, wird das Haarbueschel 1 mit dem ringfoermigen Wurzelersatz 2, der steif-elastische Merkmale aufweist, waehrend des recht einfachen Eingriffes in die Kopfhaut (4 ca. 5 mm tief) eingebracht. Selbstverstaendlich ist die Einbringtiefe des ringfoermigen Wurzelersatzes 2 vom Operateur auszuwaehlen und ist abhaengig von Abmessungen des ringfoermigen Wurzelersatzes 2, sowie von der Dicke der Kopfhaut des Prothesetraegers. Um ein Haarbueschel 1 in die Kopfhaut 4 einsetzen zu koennen, wird der ringfoermige Wurzelersatz 2 an dem hakenartig ausgebildeten Ende 5 einer mit einer Gabel 6 versehenen Nadel eingefaedelt, wie dies in der Zeichnung dargestellt ist und in die Kopfhaut eingefuehrt.

Um eine teilweise oder vollstaendige Haarprothese herzustellen, wird eine Vielzahl von Haarbuescheln 1 unter Einhalten eines gewissen Abstandes von zwischen den Haarbuescheln entsprechend aesthetischen Gesichtspunkten in die Kopfhaut eingesetzt. Es ist ferner zu unterstreichen, dass auch Haarbueschel, bestehend aus einer unterschiedlichen Anzahl von Einzelhaaaren, in die Kopfhaut eingesetzt werden koennen. Die Moeglichkeit, ein Haarbueschel mit mehreren Haaren 1 zu verwenden, ermoeglicht es, mehr als ein Haar gleichzeitig als Einzelimplantat einzusetzen. Dies weist erhebliche Zeitersparung gegenueber den Implantatverfahren auf, die ausschliesslich Einzelhaare verwenden.

Das an der Kopfhaut hervorgerufene Trauma wird ferner bei Einsetzen zahlreicher Haare wesentlich vermindert, und die Infektionsgefahr wird erheblich eingedaemmt.

Die vom Chirurgen vorzunehmende Handarbeit wird bei gleichbleibendem, aesthetischen Endergebnis wesentlich vermindet.

Wie bereits dargestellt, findet in vorteilhafter Weise eine Nadel 5 Verwendung, die ein gabelartiges Ende 6 aufweist, wobei an dieser Gabe 6 das ringfoermig ausgebildete Ende des Wurzelersatzes 2 des Haarbueschels 1 aufgesteckt wird. Unter Vorwaertsbewegen der Nadel in Richtung des Pfeiles (f) wird der ringartige steif-elastisch ausgebildete Wurzelersatz 2 in die Kopfhaut eingefuehrt.

In vorteilhafter Weise ist der Durchmesser der Nadel 5 wesentlich kleiner gewaehlt, als der Durchmesser des ringartigen Wurzelersatzes 2. So weist z.B. die Nadel 5 einen Durchmesser von 0,3 - 0,6 mm auf. Aufgrund dieses Massunterschiedes und unter Verwendung eines ringfoermigen Wurzelersatzes 2, der wesentlich groesser ist und z.B. einen Durchmesser von 0,5 - 2,0 mm aufweist, erfolgt waehrend des Einsetzens des Wurzelersatzes 2 in die Kopfhaut 4 eine momentane, elastische Verformung des ringfoermigen Implantats 2. Dieser wird zusammengedrueckt und, nach Einfuegen in die Kopfhaut 4 tendiert das ringfoermige Endestueck dazu aufgrund seiner natuerlichen Elastizitaet, erneut seinen urspruenglichen, groesseren Durchmesser einzunehmen, wodurch die Haare 1 des Bueschels fest von dem sehr kleinen Kanal umgeben werden. Dieser Kanal schliesst sich nach Ausziehen der Nadel 5 erneut und somit ist bereits ein Festlegen der Prothese aufgrund des mechanischen Druckes erreicht, der durch die Waende des Kanales in der Kopfhaut auf die Haare 1 uebertragen wird. Das Risiko einer Infektion wird wesentlich vermindert, und es tritt bald ein Vernarben der Kopfhaut ein.

Der ringfoermig ausgebildete Wurzelersatz, der einen wesentlich groesseren Druchmesser als der Einfuehrungskanal aufweist, weitet sich, sofort nach Einfuehren in die Kophaut, aufgrund der steif-elastischen Merkmale des Ringes 2 (dank der Aufbringung des Inertklebers) erneut aus. Somit wird dem ringfoermigen Wurzelersatz die Moeglichkeit genommen, ueber den wesentlich engeren Einfuehrkanal erneut aus der Kopfhaut auszutreten. Sofort nach Einsetzen des Haarbueschels, wird dieses aufgrund der mechanischen Zusammenpresswirkung sicher in der Kopfhaut gehalten.

Der ringfoermige Wurzelersatz 2 wird aufgrund des Vernarbungsvorganges vom Kopfhautgewebe durchdrungen. Das Kopfhautgewebe durchdringt waehrend des Vernarbungsvorganges vollstaendig den ringfoermigen Wurzelersatz 2, der sich in aufgeweiteter Lage befindet (aufgrund der steif-elastischen Merkmale des Ringes) und mit Durchdringen des ringfoermigen Wurzelersatzes 2 durch das Kopfhautgewebe wird nach erfolgter Vernarbung das Haarbueschel 1 und die ringfoermige Wurzel 2 fest in der Kopfhaut verankert.

**Patentansprüche**

1. Haarprothese zur Herstellung eines teilweisen oder vollstaendigen Haarersatzes mittels Implantats aus Naturhaaren, **dadurch gekennzeichnet**, dass ein oder mehrere Naturhaare (1) an ihrem in die Kopfhaut einzupflanzenden, der natuerlichen Wurzel der Haare entsprechenden, Ende zu einem ringfoermigen Gebilde (2) zusammengefuegt sind und diesem durch gegenseitiges Umwickeln und/oder Verflechten und anschliessendes Aufbringen eines Inertklebers und Abschneiden der abstehenden Endstuecke (3) der Haarenden die Gestalt eines glatten, steif-elastischen Ringes verliehen wird, wobei das ringfoermige Wurzelende (2) durch Aufbringen eines Inertklebers gefestigt wird.

2. Prothese, nach Patentanspruch 1, **dadurch gekennzeichnet**, dass der Kleber aus Zyan-Acrylat besteht.

3. Prothese, nach Patentanspruch 1, **dadurch gekennzeichnet**, dass das Haarbueschel aus natuerlichen Haaren mit unterschiedlicher Faerbung und unterschiedlicher Haardicke besteht.

4. Prothese, nach Patentanspruch 1, **dadurch gekennzeichnet**, dass der Durchmesser des ringfoermigen Wurzelendes (2) einem Wert von 0,5 - 2 mm entspricht.

**Claims**

1. Hair prosthesis for the creation of a partial or complete hair substitute by means of an implant made from natural hairs, characterised in that one or more natural hairs (1) are joined together, at their end to be implanted into the scalp and corresponding to the natural root of the hairs, to form an annular structure (2) and this structure, is given the form of a smooth, stiffly elastic ring as a result of mutual wrapping and/or intertwinement and the subsequent application of an inert adhesive and the trimming of the protruding end pieces (3) of the hair ends, the annular root end (2) being fastened by the application of an inert adhesive.

2. Prosthesis according to Patent Claim 1, characterised in that the adhesive consists of cyanacrylate.

3. Prosthesis according to Patent Claim 1, characterised in that the hair tuft consists of natural hairs of different colouring and different hair thickness.

4. Prosthesis according to Patent Claim 1, characterised in that the diameter of the annular root end (2) has a value of 0.5 - 2 mm.

**Revendications**

1. Prothèse capillaire pour fabriquer un substituant partiel ou total de cheveux au moyen d'un implant constitué de cheveux naturels, caractérisé en ce qu'un ou plusieurs cheveux naturels (1) sont réunis à leur extrémité correspondant à la racine naturelle des cheveux et à implanter dans la peau du crâne, pour former une configuration annulaire (2) et qu'à celle-ci est conférée, par enroulement mutuel et/ou tressage et application suivante d'une colle inerte et par coupe des parties extrêmes en saillie (3) des extrémités de cheveux, la forme d'un anneau lisse, rigide-élastique, l'extrémité de la racine annulaire (2) étant renforcée par application d'une colle inerte.

2. Prothèse selon la revendication 1, caractérisée en ce que la colle est en acrylate de cyanogène.

3. Prothèse selon la revendication 1, caractérisée en ce que la touffe de cheveux comprend des cheveux naturels de colorations différentes et d'épaisseurs différentes.

4. Prothèse selon la revendication 1, caractérisée en ce que le diamètre de l'extrémité de racine annulaire (2) présente une valeur de 0,5 - 2 mm.

FIG.1

FIG.2

FIG.3

FIG. 4

FIG. 5

FIG.6